# EUROPÄISCHE PATENTSCHRIFT

(11) **EP 1 900 465 B1**
(45) Veröffentlichungstag und Bekanntmachung des Hinweises auf die Patenterteilung: **02.04.2014**
(21) Anmeldenummer: 07017589.8
(22) Anmeldetag: 07.09.2007
(51) Int. Cl.: B23D 51/02

(54) **Vorrichtung zur Führung eines Sägeblattes**
Device for guiding a saw blade
Dispositif destiné au guidage d'une lame de scie

(30) Priorität: 15.09.2006 DE 202006014895 U
(43) Veröffentlichungstag der Anmeldung: 19.03.2008
(73) Patentinhaber: C. & E. Fein GmbH, 73529 Schwäbisch-Gmünd-Bargau (DE)
(72) Erfinder: Thomaschewski, Walter, 70794 Filderstadt (DE)
(74) Vertreter: Gahlert, Stefan

(56) Entgegenhaltungen:
- EP-A- 0 466 659
- FR-A- 2 590 159
- US-A- 1 381 033
- US-A- 5 925 049
- US-A1- 2003 236 524
- US-A1- 2004 260 301
- US-A1- 2005 228 393
- US-A1- 2005 240 196
- US-A1- 2006 111 725
- US-B1- 6 458 135

## Beschreibung

Die Erfindung betrifft eine Vorrichtung zur Führung eines Sägeblattes, insbesondere für ein oszillierend angetriebenes Sägeblatt, mit einem Grundkörper, der von einem Führungsschlitz zur Führung eines Sägeblattes durchsetzt ist, der in eine erste Führungsfläche zur Anlage an einem Werkstück ausmündet, mit einem am Grundkörper gehaltenen Anschlag, der eine zweite Führungsfläche zur Anlage am Werkstück aufweist, die winklig zur ersten Führungsfläche angeordnet ist, und mit einem Handgriff zum Halten des Grundkörpers.

Eine derartige Vorrichtung zur Führung eines Sägeblattes ist aus der FR-A-2590159 bekannt.

Bei der bekannten Vorrichtung handelt es sich um eine Schnittlehre, die zur Implantierung eines künstlichen Kniegelenkes an einem Knie verwendet wird. Die Lehre ist genau auf die Erfordernisse beim Implantieren eines künstlichen Kniegelenkes abgestimmt.

Vorrichtungen ähnlicher Art sind aus der US-A-5925049, der US-B1-6458135, der EP-A-0466659, der US 2004/0260301 A1 und aus der US-A-1381033 bekannt.

Die bekannten Vorrichtungen zur Führung von Sägeblättern, die überwiegend bei der Implantierung von künstlichen Kniegelenken verwendet werden, erlauben eine genaue Adaption an die örtlichen Gegebenheiten bei der Implantierung und an die präzise Einstellung, in der Regel unter Verwendung von Fixierungselementen zur temporären Fixierung ans Kniegelenk.

Aus der US 2005/0228393 A1 ist ferner ein Schneidblock mit einer Führung zur Verwendung bei der Implantation von künstlichen Kniegelenken bekannt, der aus einem Kunststoffmaterial mit metallischen Verstärkungen hergestellt ist.

Vor diesem Hintergrund liegt der Erfindung die Aufgabe zugrunde, eine verbesserte Vorrichtung zur Führung eines Sägeblattes zu offenbaren, die auf möglichst einfache und kostengünstige Weise hergestellt und in einfacher Weise gehandhabt werden kann.

Diese Aufgabe wird bei einer Vorrichtung gemäß der eingangs genannten Art erfindungsgemäß dadurch gelöst, dass der Handgriff am Grundkörper seitlich abgekröpft aufgenommen ist und als einstückig mit dem Grundkörper verbundenes Kunststoffteil ausgebildet ist, und dass der Führungsschlitz in einem metallischen Einsatz am Grundkörper ausgebildet ist.

Die Aufgabe der Erfindung wird auf diese Weise vollkommen gelöst.

Mit der erfindungsgemäßen Vorrichtung ist ein sehr komfortables Halten mittels des seitlich abgekröpft am Grundkörper aufgenommenen Handgriffes ermöglicht. Gleichzeitig ergibt sich eine einfache und kostengünstige Herstellung, da der Handgriff und der Grundkörper als einstückiges Kunststoffteil ausgebildet sind. Ferner ist eine präzise Führung des Sägeblattes dadurch gewährleistet, dass der Führungsschlitz in einem metallischen Einsatz am Grundkörper ausgebildet ist.

Mit der erfindungsgemäßen Vorrichtung lässt sich ein Führungsschlitz zur Führung eines Sägeblattes mit mindestens zwei Führungsflächen an einem Werkstück präzise positionieren. Durch den Führungsschlitz ist der Ort des hierdurch in das Werkstück eindringenden Sägeblattes genau vorgegeben. Auf diese Weise können Schlitze in Werkstücke an vorbestimmten Positionen präzise erzeugt werden.

In vorteilhafter Weiterbildung der Erfindung ist der Anschlag verschiebbar am Grundkörper aufgenommen.

Auf diese Weise kann der Abstand zwischen dem durch den Führungsschlitz geführten Sägeblatt und der zweiten Führungsfläche einstellbar ausgebildet sein, um so den Abstand des zu erzeugenden Schlitzes von einer Werkstückoberfläche einstellbar zu machen.

Die zweite Führungsfläche ist vorzugsweise rechtwinklig zur ersten Führungsfläche angeordnet.

Auf diese Weise ergibt sich eine besonders einfache Handhabung, da die Vorrichtung so mit den beiden rechtwinklig zueinander angeordneten Führungsflächen an eine rechtwinklige Außenkante eines Werkstückes angesetzt werden kann.

Grundsätzlich wäre es jedoch auch denkbar, den Winkel zwischen den beiden Führungsflächen einstellbar zu machen, sofern eine Bearbeitung von nicht rechtwinkligen Werkstücken gewünscht ist.

Gemäß einer weiteren bevorzugten Ausgestaltung der Erfindung ist der Anschlag als Winkel ausgebildet, der mit einem Führungselement an einer Aufnahme des Grundkörpers verschiebbar geführt ist.

Auf diese Weise ergibt sich ein einfacher Aufbau und eine einfache Verstellmöglichkeit des Abstandes zwischen der zweiten Führungsfläche und dem Führungsschlitz.

In zusätzlicher Weiterbildung dieser Ausbildung ist das Führungselement an der Aufnahme mittels Spannelementen feststellbar gehalten.

Auf diese Weise kann ein vorbestimmter Abstand zwischen einer Werkstückoberfläche und dem zu erzeugenden Schlitz vor Beginn des Schnittes eingestellt und fixiert werden.

Hierzu können die Spannelemente Schrauben aufweisen, die den Grundkörper durchsetzen und in Gewindestücke eingreifen, durch die das Führungselement gegen die Aufnahme anpressbar ist.

Auf diese Weise ergibt sich eine besonders einfache Verstellmöglichkeit für den Anschlag.

Gemäß einer weiteren bevorzugten Ausgestaltung der Erfindung ist der Anschlag als Kunststoffteil, vorzugsweise als Spritzgussteil, ausgeführt.

Hierdurch ergibt sich eine einfache und kostengünstige Herstellung des Anschlages.

Gemäß einer weiteren bevorzugten Ausgestaltung der Erfindung ist am Grundkörper eine Skala vorgesehen, an der der Abstand zwischen dem Schlitz und der zweiten Führungsfläche ablesbar ist.

Durch dieses Merkmal wird eine Voreinstellung des Abstandes zwischen der von dem Anschlag gebildeten zweiten Führungsfläche und dem Führungsschlitz erleichtert.

Gemäß einer weiteren bevorzugten Ausgestaltung der Erfindung ist die erste Führungsfläche am Grundkörper von mindestens einer Ausnehmung durchsetzt.

Auf diese Weise kann der Grundkörper beispielsweise mittels einer Schraube zuvor an einem Werkstück fixiert und somit sicher positioniert werden, bevor mit dem Sägevorgang begonnen wird.

Gemäß einer weiteren bevorzugten Ausgestaltung der Erfindung ist die Führungsfläche am Anschlag von mindestens einer Ausnehmung durchsetzt.

Auf diese Weise kann die Vorrichtung vor Beginn eines Schnittes alternativ oder zusätzlich mittels des Anschlages am Werkstück fixiert werden, etwa mit Hilfe einer Schraube. Hierzu kann die zweite Führungsfläche am Anschlag etwa von einem oder mehreren Schlitzen durchsetzt sein.

Es versteht sich, dass die vorstehend genannten und die nachstehend noch zu erläuternden Merkmale der Erfindung nicht nur in der jeweils angegebenen Kombination, sondern auch in anderen Kombinationen oder in Alleinstellung verwendbar sind, ohne den Rahmen der vorliegenden Erfindung zu verlassen.

Weitere Merkmale und Vorteile der Erfindung ergeben sich aus der nachfolgenden Beschreibung eines bevorzugten Ausführungsbeispiels unter Bezugnahme auf die Zeichnung. Es zeigen:
- Figur 1: eine Seitenansicht einer erfindungsgemäßen Vorrichtung, wobei zusätzlich ein Oszillationsantrieb mit einem Sägeblatt angedeutet ist, das durch den Führungsschlitz der Vorrichtung hindurch geführt ist und in dieser Stellung zur Erzeugung eines schlitzförmigen Schnittes im Werkstück verwendet werden kann und
- Figur 2: eine Ansicht der Vorrichtung gemäß Figur 1 von unten.

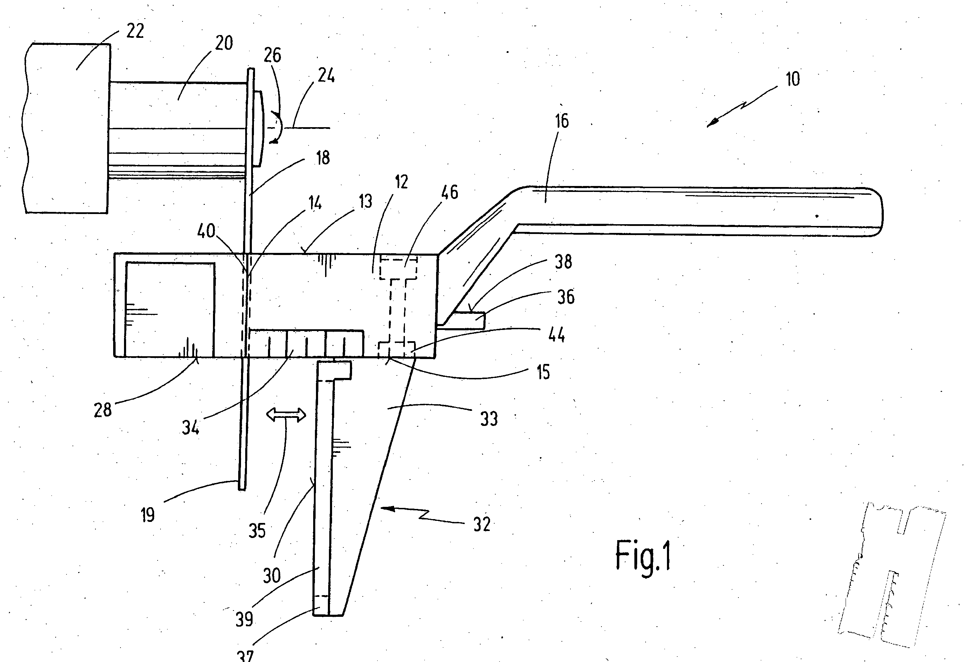

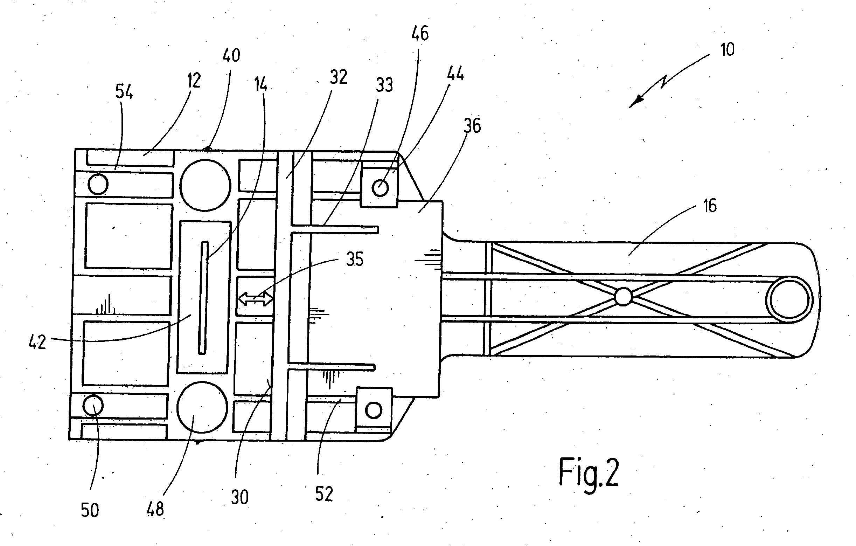

In den Figuren 1 und 2 ist eine erfindungsgemäße Vorrichtung dargestellt und insgesamt mit der Ziffer 10 bezeichnet.

Die Vorrichtung 10 weist einen annähernd quaderförmigen Grundkörper 12 auf, der, wie aus Figur 2 zu ersehen ist, jedoch nicht als Massivkörper, sondern als Hohlprofilkörper ausgestaltet ist. Hierdurch ergibt sich eine gewichts- und materialsparende Ausführung.

Der Grundkörper wird mittels eines seitlich daran abgekröpft aufgenommenen Handgriffes 16 gehalten. Der Grundkörper 12 weist eine erste Oberfläche 13 an der Oberseite und eine zweite Oberfläche 15 an der Unterseite auf. Zwischen der ersten Oberfläche und der zweiten Oberfläche erstreckt sich ein rechteckförmiger Führungsschlitz 14, der zur Führung eines hindurchgesteckten Werkzeuges 18 dient.

Das Werkzeug 18 ist im dargestellten Fall als längliches Sägeblatt ausgebildet, mit einer Schneide 19 am unteren Ende, das am der Schneide gegenüberliegenden Ende auf einer Antriebswelle 20 eines Oszillationsantriebes 22 befestigt ist.

Der Oszillationsantrieb 22 versetzt die Antriebswelle 20 in eine hin- und hergehende Oszillationsbewegung um die Längsachse 24 der Antriebswelle, wie durch den Doppelpfeil 26 angedeutet ist. Hierbei erfolgt der Antrieb in der Regel mit hoher Frequenz zwischen etwa 5.000 und 30.000 Oszillationen pro Minute und mit kleinem Verschwenkwinkel zwischen 0,5 und 7 Grad.

Mit einem solchermaßen oszillierend angetriebenen Sägeblatt 18 können schlitzförmige Schnitte in zahlreichen Werkstücken erzeugt werden.

Hierbei dient die erfindungsgemäße Vorrichtung zur präzisen Positionierung eines Schnittes und dem rechtwinkligen Eintauchen des Sägeblatts an einem Werkstück.

Die zweite, untere Oberfläche 15 des Grundkörpers 12 ist als eine erste Führungsfläche 28 ausgebildet, die zur Führung der Vorrichtung entlang einer Werkstückoberfläche dient.

Am Grundkörper 12 ist ferner ein rechtwinklig ausgebildeter Anschlag 32 gehalten, der mit einer Anschlagplatte 37 rechtwinklig von der ersten Führungsfläche 28 absteht. Durch die Oberfläche der Anschlagplatte 37 ist eine zweite Führungsfläche 30 gebildet.

Da der Anschlag 32, wie nachfolgend noch anhand von Figur 2 näher erläutert wird, parallel zur ersten Führungsfläche 28 verstellbar ist (vgl. Pfeil 35), kann der Abstand zwischen der zweiten Führungsfläche 30 und dem Führungsschlitz 14 eingestellt werden.

Um die Einstellung zu erleichtern, ist an beiden Seitenflächen des Grundkörpers 12 eine Markierung 40 vorgesehen, die die Mitte des Führungsschlitzes 14 kennzeichnet, und von der ausgehend sich eine Skala 34 erstreckt.

Der Anschlag 32 ist durch zwei Versteifungsrippen 33, die sich von der Anschlagplatte 37 aus bis zu einer rechtwinklig zur Anschlagplatte 37 erstreckenden Platte 36 erstrecken, versteift. Die Platte 36 dient als Führungselement zur Führung an einer Aufnahme 52 (Fig. 2). Der Anschlag 32 kann somit durch Verschiebung des als Platte ausgebildeten Führungselementes 36 entlang der Aufnahme erstellt werden, wie durch den Pfeil 35 angedeutet ist.

Zur Fixierung des Anschlages in einer einmal eingestellten Position dienen Gewindestücke 44 in Form von Plättchen, die an der Unterseite des Grundkörpers aufgenommen sind, und das Führungselement 36 teilweise seitlich überdecken. Die Gewindestücke 44 sind durch Schrauben 46 gehalten, die von der ersten Oberfläche 13 an der Oberseite des Grundkörpers 12 durch diesen hindurch geschraubt sind und in die Gewindestücke 44 eingreifen. So kann die Feststellung des Anschlages 32 in einer gewünschten Position am Grundkörper durch Lösen der Schrauben 46 gelöst und der Anschlag 32 nach erfolgter Einstellung durch Anziehen der Schrauben 34 fixiert werden. So kann eine Voreinstellung des Abstandes zwischen der zweiten Führungsfläche 30 und dem Führungsschlitz 14 vor der Erzeugung eines Schnittes erfolgen.

Der Grundkörper 12 ist von einer Mehrzahl von Ausnehmungen 48, 50 durchsetzt, die zur Fixierung des Grundkörpers 12 vor der Erzeugung eines Schnittes an einem Werkstück dienen können oder alternativ auch als Bohrlehre bei der Erzeugung von Bohrungen am Werkstück. Sollten die Ausnehmungen zur Erzeugung von Bohrungen verwendet werden, so können in diese vorzugsweise Metalleinsätze eingesetzt werden, um verschiedene Bohrungsdurchmesser zu ermöglichen und um eine vorzeitige Abnutzung zu verhindern.

Der Grundkörper 12 und der Handgriff 16 sind einstückig als Kunststoffspritzgussteil ausgebildet mit einer durchgehenden Oberfläche an der Seite 13 und mit einer Gestaltung als Hohlprofil, dessen Unterseite 15 lediglich durch eine Reihe von Stegen 54 festgelegt ist, die sich nach unten hin erstrecken und somit die erste Führungsfläche 28 definieren. Der Führungsschlitz 14 ist in einem aus Metall, vorzugsweise aus Stahl, bestehenden Einsatz 42 ausgebildet.

Auch der Anschlag 32 ist als Kunststoffspritzgussteil hergestellt.

Die Anschlagplatte 37 kann zusätzlich von einer oder mehreren Ausnehmungen 39 durchsetzt sein, beispielsweise in Form von drei Schlitzen, die sich parallel zu den seitlichen Außenoberflächen in der Anschlagplatte 37 von oben nach unten erstrecken.

Diese Schlitze dienen einerseits der Gewichtsersparnis und können andererseits zur kurzzeitigen Fixierung der Vorrichtung 10 an einer Werkstückoberfläche vor Erzeugung eines Schnittes genutzt werden, indem z.B. eine Schraube eingedreht wird.

## Patentansprüche

1. Vorrichtung zur Führung eines Sägeblattes, insbesondere für ein oszillierend angetriebenes Sägeblatt, mit einem Grundkörper (12), der von einem Führungsschlitz (14) zur Führung eines Sägeblattes (18) durchsetzt ist, der in eine erste Führungsfläche (28) zur Anlage an einem Werkstück ausmündet, mit einem am Grundkörper (12) gehaltenen Anschlag (32), der eine zweite Führungsfläche (30) zur Anlage am Werkstück aufweist, die winklig zur ersten Führungsfläche (28) angeordnet ist, und mit einem Handgriff (16) zum Halten des Grundkörpers (12), **dadurch gekennzeichnet, dass** der Handgriff (16) am Grundkörper (12) seitlich abgekröpft aufgenommen ist und als einstückig mit dem Grundkörper (12) verbundenes Kunststoffteil ausgebildet ist, und dass der Führungsschlitz (14) in einem metallischen Einsatz (42) am Grundkörper (12) ausgebildet ist.

2. Vorrichtung nach Anspruch 1, bei der der Anschlag (32) verschiebbar am Grundkörper (12) aufgenommen ist.

3. Vorrichtung nach Anspruch 1 oder 2, bei der die zweite Führungsfläche (30) rechtwinklig zur ersten Führungsfläche (28) angeordnet ist.

4. Vorrichtung nach einem der vorhergehenden Ansprüche, bei der der Anschlag als Winkel (32) ausgebildet ist, der mit einem Führungselement (36) an einer Aufnahme (52) des Grundkörpers (12) verschiebbar geführt ist.

5. Vorrichtung nach Anspruch 4, bei der das Führungselement (36) an der Aufnahme (52) mittels Spannelementen (44, 46) feststellbar gehalten ist.

6. Vorrichtung nach Anspruch 5, bei der die Spannelemente Schrauben (46) aufweisen, die den Grundkörper durchsetzen und in zugeordnete Gewindestücke (44) eingreifen, durch die das Führungselement (36) gegen die Aufnahme (52) anpressbar ist.

7. Vorrichtung nach einem der vorhergehenden Ansprüche, **dadurch gekennzeichnet, dass** der Anschlag (32) als Kunststoffteil ausgebildet ist, das vorzugsweise als Spritzgussteil ausgeführt ist.

8. Vorrichtung nach einem der Ansprüche 4 bis 7, bei der am Grundkörper (12) eine Skala (34) vorgesehen ist, an der der Abstand zwischen dem Schlitz (14) und der zweiten Führungsfläche (30) ablesbar ist.

9. Vorrichtung nach einem der vorhergehenden Ansprüche, bei der die erste Führungsfläche (28) am Grundkörper (12) von mindestens einer Ausnehmung (48, 50) durchsetzt ist.

10. Vorrichtung nach einem der vorhergehenden Ansprüche, bei der die zweite Führungsfläche (30) am Anschlag (32) von mindestens einer Ausnehmung durchsetzt ist.

## Claims

1. A device for guiding a saw blade, in particular a saw blade that is driven in an oscillating way, having a base body (12) through which a guide slot (14) extends for guiding a saw blade (18), which guide slot opens into a first guide surface (28) intended as contact surface for the workpiece, having a stop (32) which is supported on the base body (12) and which comprises a second guide surface (30) intended for being brought into contact with the workpiece and arranged at an angle relative to the first guide surface (28), and having a handle (16) as means for holding the base body (12), **characterized in that** the handle (16) is received on the base body (12) laterally cranked and is configured as a plastic part integral with the base body (12), and **in that** the guide slot (14) is formed in a metallic insert (42) on the base body (12).

2. The device of claim 1, wherein the stop (32) is received on the base body (12) in sliding arrangement.

3. The device of claim 1 or 2, wherein the second guide surface (30) is arranged at a right angle relative to the first guide surface (28).

4. The device of any of the preceding claims, wherein the stop is configured as an angle (32) that is slidably guided on a seat (52) of the base body (12) by a guide element (36).

5. The device of claim 4, wherein the guide element (36) is held on the seat (52) and can be fixed in place by clamping elements (44, 46).

6. The device of claim 5, wherein the clamping elements comprise screws (46) that pass through the base body and engage coupling pieces (44) by means of which the guide element (36) can be urged against the seat (52).

7. The device of any of the preceding claims, wherein the stop (32) is configured as a plastic part, preferably as an injection-molded part.

8. The device of any of claims 4 to 7, wherein a scale (34) is provided on the base body (12) for indicating the spacing between the slot (14) and the second guide surface (30).

9. The device of any of the preceding claims, wherein the first guide surface (28) on the base body (12) is passed by at least one opening (48, 50).

10. The device of any of the preceding claims, wherein the second guide surface (30) on the stop (32) is passed by at least one opening.

## Revendications

1. Dispositif destiné au guidage d'une lame de scie, en particulier pour une lame de scie entraînée de manière oscillante, comprenant un corps de base (12) traversé d'une fente de guidage (14) pour guider une lame de scie (18), qui débouche dans une première surface de guidage (28) de manière à venir en appui contre une pièce, comprenant une butée (32) maintenue sur le corps de base (12), laquelle présente une deuxième surface de guidage (30) pour venir en butée contre la pièce, laquelle est disposée de manière inclinée par rapport à la première surface de guidage (28), et comprenant une poignée (16) pour retenir le corps de base (12), **caractérisé en ce que** la poignée (16) est reçue de manière coudée latéralement sur le corps de base (12) et est réalisée sous forme de pièce en plastique connectée d'une seule pièce au corps de base (12) et **en ce que** la fente de guidage (14) est réalisée dans un insert métallique (42) sur le corps de base (12).

2. Dispositif selon la revendication 1, dans lequel la butée (32) est reçue de manière déplaçable sur le corps de base (12).

3. Dispositif selon la revendication 1 ou 2, dans lequel la deuxième surface de guidage (30) est disposée à angle droit par rapport à la première surface de guidage (28).

4. Dispositif selon l'une quelconque des revendications précédentes, dans lequel la butée est réalisée sous forme de coude (32) qui est guidé de manière déplaçable avec un élément de guidage (36) contre un logement (52) du corps de base (12).

5. Dispositif selon la revendication 4, dans lequel l'élément de guidage (36) est maintenu de manière à pouvoir être fixé sur le logement (52) au moyen d'éléments de serrage (44, 46).

6. Dispositif selon la revendication 5, dans lequel les éléments de serrage présentent des vis (46) qui traversent le corps de base et qui viennent en prise dans des pièces filetées associées (44) par lesquelles l'élément de guidage (36) peut être pressé contre le logement (52).

7. Dispositif selon l'une quelconque des revendications précédentes, **caractérisé en ce que** la butée (32) est réalisée sous forme de pièce en plastique qui est réalisée de préférence sous forme de pièce moulée par injection.

8. Dispositif selon l'une quelconque des revendications 4 à 7, dans lequel une échelle (34) est prévue sur le corps de base (12), sur laquelle peut être lue la distance entre la fente (14) et la deuxième surface de guidage (30).

9. Dispositif selon l'une quelconque des revendications précédentes, dans lequel la première surface de guidage (28) sur le corps de base (12) est traversée d'au moins un évidement (48, 50).

10. Dispositif selon l'une quelconque des revendications précédentes, dans lequel la deuxième surface de guidage (30) sur la butée (32) est traversée d'au moins un évidement.
